# EUROPÄISCHE PATENTSCHRIFT

(11) **EP 1 105 141 B1**
(45) Veröffentlichungstag und Bekanntmachung des Hinweises auf die Patenterteilung: **05.11.2003**
(21) Anmeldenummer: 99952284.0
(22) Anmeldetag: 06.08.1999
(51) Int. Cl.: A61K 31/685, A61P 1/00

(54) **PHOSPHATIDYLCHOLIN ALS ARZNEIMITTEL MIT SCHLEIMHAUTSCHÜTZENDER WIRKUNG**
PHOSPHATIDYLCHOLINE AS A MEDICAMENT FOR THE PROTECTION OF MUCOSA
PHOSPHATIDYLCHOLINE EN TANT QUE MEDICAMENT A ACTION PROTECTRICE DES MUQUEUSES

(30) Priorität: 06.08.1998 DE 19835526; 15.12.1998 DE 19857750
(43) Veröffentlichungstag der Anmeldung: 13.06.2001
(73) Patentinhaber: Stremmel, Wolfgang, 69120 Heidelberg (DE)
(72) Erfinder: Stremmel, Wolfgang, 69120 Heidelberg (DE)
(74) Vertreter: Naumann, Ulrich, Dr.-Ing.
(86) Internationale Anmeldenummer: DE9902426
(87) Internationale Veröffentlichungsnummer: WO00007577

(56) Entgegenhaltungen:
- WO-A-95/26646
- DATABASE WPI Section Ch, Week 198616 Derwent Publications Ltd., London, GB; Class B04, AN 1986-103236 XP002144293 & JP 61 047418 A (SEIKAGAKU KOGYO CO LTD), 7. März 1986 (1986-03-07)
- R. FABIA: "Effects of phosphatidycholine and phosphatidylinositol on acetic-acid induced colitis in the rat" DIGESTION, Bd. 53, Nr. 1-2, 1992, Seiten 35-44, XP000091107
- M. MOURELLE: "Polyunsaturated phosphatidylcholine prevents stricture formation in a rat model of colitis" GASTROENTEROLOGY, Bd. 110, Nr. 4, 1996, Seiten 1093-1097, XP000907314
- SMITH A J ET AL: "The human MDR3 P-glycoprotein promotes translocation of phosphatidylcholine through the plasma membrane of fibroblasts from transgenic mice." FEBS LETTERS, (1994 NOV 14) 354 (3) 263-6, XP002142062
- DE VREE J M ET AL: "Mutations in the MDR3 gene cause progressive familial intrahepatic cholestasis." PROCEEDINGS OF THE NATIONAL ACADEMY OF SCIENCES OF THE UNITED STATES OF AMERICA, (1998 JAN 6) 95 (1) 282-7, XP002142063
- VAN HELVOORT A ET AL: "MDR1 P-glycoprotein is a lipid translocase of broad specificity, while MDR3 P-glycoprotein specifically translocates phosphatidylcholine." CELL, (1996 NOV 1) 87 (3) 507-17, XP002142064
- PANWALA C M ET AL: "A novel model of inflammatory bowel disease: mice deficient for the multiple drug resistance gene, mdr1a, spontaneously develop colitis." JOURNAL OF IMMUNOLOGY, (1998 NOV 15) 161 (10) 5733-44., XP002142065

## Beschreibung

Die vorliegende Erfindung betrifft die Herstellung Arzneimittel enthaltend als Wirkstoff Phosphatidylcholin in einer therapeutisch wirksamen Menge zur Behandlung von Erkrankungen, bei denen die schleimhautschützende Wirkung von Phophatidylcholin im Dickdarm und terminalen Ileum (Pouchmucosa) vorteilhaft ist. Die Arzneimittel eigenen sich zur Behandlung von Colitis ulcerosa, Pouchitis, anderen Dickdarmentzündungen (Morbus Crohn, Diversionscolitis, infektiöse Enteritis/Colitis, Entzündungen durch Bestrahlung, Antibiotika, Chemotherapeutica, Pharmaka oder Chemikalien) sowie zur Prophylaxe des Dickdarmcarcinoms.

Die chronisch entzündlichen Darmerkrankungen Colitis ulcerosa und Morbus Crohn betreffen in hohem Maße Menschen im jugendlichen und mittleren Lebensalter mit steigender Frequenz (Prävalenz zusammen 1 - 2 %). Der chronische Verlauf mit akuten entzündlichen Schüben sowie zahlreiche Komplikationen (Fistel- und Abszeßbildungen, Stenosen, akute entzündliche Schübe, Blutungen, Funktionsverlust des Dickdarmepithels, extraintestinale Manifestation) kennzeichnen den natürlichen Verlauf dieser Erkrankungen. Insbesondere bei Colitis ulcerosa ist nach langjährigem Verlauf ein erhöhtes Risiko für die Entstehung eines Dickdarmcarcinoms hervorzuheben. Trotz intensiver Forschungsarbeiten ist es bisher nicht gelungen, die Ursache dieser Erkrankungen festzustellen. Deshalb existiert auch nur eine symptomatische Therapie, die nicht ursachenspezifisch ist und häufig nicht die gewünschten Erfolge bewirkt.

Zwischen der Colitis ulcerosa und dem Morbus Crohn bestehen wesentliche Unterschiede, so daß von zwei verschiedenen Entstehungsmechanismen ausgegangen werden kann. Der Morbus Crohn kann prinzipiell den gesamten Gastrointestinaltrakt betreffen (häufigste Lokalisation am Ende des Dünndarmes im terminalen lleum). Die entzündlichen Veränderungen sind umschrieben, in einer sonst gesunden Schleimhaut angeordnet und können mit der Zeit wechseln. Hauptkomplikationen sind entzündliche Engstellungen von Darmabschnitten sowie Fistel- und Abszeßbildungen. Manifestationen außerhalb des Gastrointestinaltraktes sind möglich. Die Colitis ulcerosa zeigt dagegen eine kontinuierliche Entzündung mit oberflächlichen Ulcerationen ausgehend vom Ende des Dickdarmes (Proktum, Rektum). Je nach Schweregrad kann sich die Colitis nach oben ausdehnen und schließlich den gesamten Dickdarm betreffen. In einem hohen Prozentsatz ist auch das Ende des Dünndarmes entzündet ("Backwash-lleitis"). Hauptkomplikationen sind Funktionseinschränkungen des Dickdarmes mit zahlreichen Durchfällen, Blutungen aus Schleimhautgeschwüren und selten eine dramatische Entzündung der gesamten Darmwand (toxisches Megacolon). Gefürchtet ist die häufige Entstehung eines Dickdarmcarcinoms nach langjährigem Krankheitsverlauf. Zwischen beiden chronisch entzündlichen Darmerkrankungen gibt es allerdings fließende Übergänge in der Symptomatik, so daß die Unterscheidung oft sehr schwierig ist.

Neben der oft unzureichenden symptomatischen Therapie muß bei der Colitis ulcerosa aufgrund des komplizierten, schweren Krankheitsbildes und der drohenden Gefahr eines Carcinoms häufig der gesamte Dickdarm entfernt werden. Dabei wird aus der letzten Dünndarmschlinge ein Reservoir (Pouch) gebildet, in den Analkanal gelegt, dort fixiert und mit dem innen ausgeschälten, natürlichen Anus verbunden. Damit ist ein neues Auffangorgan für den Darminhalt geschaffen mit Erhalt des natürlichen Ausganges (kontinenzerhaltende ileoanale Pouchanlage). Bei ca. 30% der Patienten mit Colitis ulcerosa kann sich der Pouch entzünden (Pouchitis) und zu erheblichen Beschwerden führen. Wenn eine solche Pouchanlage bei anderen Grunderkrankungen (z.B. familiärer adenomatöser Polyposis) vorgenommen wird, kommt es dagegen nur in Ausnahmefällen zu einer Entzündung.

WO 95/18622 beschreibt die Verwendung von Derivaten der 2-Hydroxy-5-phenylazobenzoesäure als chemoprotektive Substanz zur Behandlung von Krebserkrankungen des Colons.

JP 61047418 A offenbart Arzneimittel, die unter anderem Lecithin enthalten, zur Behandlung gastrointestinaler Beschwerden.

WO 95/26646 A beschreibt ein enterales, Soyalecithin-haltiges Ernährungsprodukt zur Behandlung von ulcerativer Colitis.

FABIA, R. et al. (Digestion. 1992, Band 53, Seiten 35-44) beschreiben einen therapeutischen Effekt von Phosphatidylcholin bei der Behandlung von Ratten mit Säure-induzierter Colitis. MOURELLE M. et al. (Gastroenterology. 1996, Band 110, Seiten 1093-1097) beschreiben die Verwendung von mehrfach ungesättigtem Phosphatidylcholin zur Behandlung von Darmstrikturen.

Aufgabe der vorliegenden Erfindung war es, weitere Arzneimittel zur Behandlung von Dickdarmerkrankungen zur Verfügung zu stellen.

Überraschenderweise wurde gefunden, daß Phophatidylcholin als schleimhautschützende Substanz des Dickdarms fungiert. Wie ein Schutzfilm überzieht Phosphatidylcholin die Darmzellen und eignet sich daher als therapeutischer Wirkstoff zur Behandlung von Colitis ulcerosa, Pouchitis, anderen Dickdarmentzündungen (Morbus Crohn, Diversionscolitis, infektiöse Enteritis/Colitis, Entzündungen durch Bestrahlung, Antibiotika, Chemotherapeutica, Pharmaka oder Chemikalien) sowie zur Prophylaxe des Dickdarmcarcinoms.

Phosphatidylcholin (PC) (synonym ist auch der Begriff Lecithin gebräuchlich), ist ein zusammengesetztes phosphorhaltiges Lipid (Phospholipid) mit folgender schematischer Strukturformel:

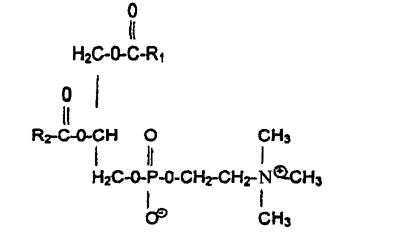

R₁ = (CH₂)ₙ-CH₃

R₂ = (CH₂)ₙ-CH₃

Zur Behandlung der Colitis ulcerosa, Pouchitis und den anderen oben genannten Dickdarmentzündungen eignen sich orale Präparationen von Phosphatidylcholin mit gezielter oder verzögerter Wirkstofffreisetzung im unteren Ileum und Colon und/oder rektale Applikationsformen, z.B. als Klysma, Suppositorium oder Schaum. Für die Prophylaxe des Coloncarcinoms, insbesondere bei Colitis ulcerosa, ist die orale Applikation bevorzugt. Die orale Gabe kann in Form von geeigneten Darreichungsformen, beispielsweise als Tabletten oder Kapseln, erfolgen, die die Resorption aus den ersten zwei Dritteln des Dünndarms verhindern und zur gezielten Freisetzung im unteren Abschnitt des Dünndarms führen.

Arzneimittel, die Phosphatidylcholin enthalten, können lokal als rektale Applikationsform (z.B. Suppositorien, Schaum oder Klysmen) oder auch oral verabreicht werden. Die Applikation von Phosphatidylcholin erfolgt in die unteren Dünndarmabschnitte und das Colon, vor allem zum Schutz der Dickdarmschleimhaut vor bakterieller Infektion. Für die orale Gabe eignen sich insbesondere solche Arzneiformen, die den Wirkstoff verzögert freisetzen (sog. Retardformen). Die Retardierung wird zweckmäßigerweise erreicht durch Überzüge und/oder Trägermatrices, die magensaftresistent sind und den Wirkstoff in Abhängigkeit des pH-Wertes im tiefen Dünndarm und Dickdarm freisetzen. Als Technismen sind Filmüberzüge geeignet, die beispielsweise Eudragit® - Acrylpolymere zur gesteuerten Wirkstoffabgabe (beispielsweise die Produkte mit der Markenbezeichnung Eudragit® L und Eudragit® S der Fa. Röhm, Darmstadt) enthalten.

Zur Herstellung von oral verabreichbarem Phosphatidylcholin wird eine verzögerte Freisetzungsform verwendet, um die Resorption in den proximalen lleumabschnitten zu vermeiden. Phosphatidylcholin kann dabei in großvolumige (z.B. 0,68 ml Inhalt) Kapseln (z.B. Gelatine) eingebracht werden. Diese sind beispielsweise mit Acrylpolymeren überzogen, z.B. Verwendung der o.g. Eugradit® -Präparaten). Eine Kombination aus Eugradit® S- und L-Verbindungen (z.B. Eugradit® L/S 100) gewährt eine verzögerte Freisetzung bei pH > 6,4, wie er im terminalen lleum vorkommt. Die Verwendung von Eugradit® Präparaten und deren Mischung (L-, S-und R-Verbindungen) ist seit langem etabliert. Ferner ist es möglich, daß auch andere Überzüge oder Darreichungsformen (auch Neuentwicklungen) zur Freisetzung im terminalen Ileum eingesetzt werden können, wenn sie sich als optimale Lösung anbieten.

Zur Herstellung der Klysmen können Phosphatidylcholin-Präparationen (Lecithin) in einem lipophilen Lösungsmittel aufgelöst werden (z.B. Sojaöl). Die zu verabreichende Dosis beträgt beispielsweise 5 - 20 g in 100 ml Sojaöl. 100 ml der Lösung können als Klysma rektal appliziert werden. Daneben sollen Darreichungsformen als Suppositorien oder Schaumpräparationen einsetzbar sein.

Erfindungsgemäße Arzneimittel enthalten Phosphatidylcholin in einer therapeutisch wirksamen Menge, die ausreicht, um die schleimhautschützende Wirkung im Dickdarm zu bewirken. Der Wirkstoffgehalt in der Fertigarzneiform beträgt 1 - 500 mg, bevorzugt 100 - 300 mg. Für die orale Gabe kommen als geeignete Arzneiformen Tabletten, Granulate, Kapseln, Pellets oder Pellettabletten in Frage. Die Arzneiformen können ferner übliche pharmazeutische Zusatzstoffe, wie Hilfs- oder Trägerstoffe enthalten. Für die rektale Anwendung kommen primär Klysmen, Schaumpräparationen, Salben, Gele, Lotionen und Suppositorien in Frage. Diese enthalten den Wirkstoff in einer Menge von 10 mg - 10 g, vorzugsweise bis zu 5 g. Je nach Schwere der Erkrankung werden die Arzneiformen ein- oder mehrfach täglich verabreicht.

Beschrieben wird ferner ein diagnostisches Verfahren zum Nachweis von Krankheiten, die mit einer verminderten Sekretion von Phosphatidylcholin in das intestinale Lumen einhergehen, wie z.B. die Colitis ulcerosa, die Pouchitis bei Colitis ulcerosa oder auch die Diversionscolitis. In diesem Zusammenhang wird auch ein Verfahren zur Bestimmung der Phosphatidylcholinkonzentration im Schleim der Colonschleimhaut beschrieben, wobei die zu analysierende Probe beispielsweise mit Hilfe eines Tupfpräparates entnommen werden kann. Anschließend wird durch eine geeignete lipidchemische Analyse, beispielsweise durch Massenspektrometrie, die Lipidzusammensetzung bestimmt.

Überraschenderweise wurde gefunden, daß als Indikator für die schleimhautschützende Wirkung des sich im Darmschleim befindlichen Phosphatidylcholins die Bestimmung von MDR3-analogen Proteinen von diagnostischer Bedeutung ist (MDR = Multi Drug Resistance). Das Fehlen von MDR3-analogen Proteinen im terminalen lleum kann in diesem Zusammenhang ein Indikator für eine unzureichende Phosphatidylcholinsekretion ins Darmlumen mit nachfolgender Veränderung der Schleimzusammensetzung im Dickdarm und gesteigerter Anfälligkeit gegenüber dort ansässigen Bakterien darstellen. Die unzureichende Produktion von MDR3-analogen Proteinen könnte so die reduzierte Menge an Phophatidylcholin im Darmlumen erklären. Im Sinne der vorliegenden Erfindung konnte gezeigt werden, daß im Mucosaepithel des terminalen Ileum oder des Pouches bei Patienten mit Colitis ulcerosa kein MDR3-analoges Protein vorhanden ist. Dadurch ist die Phosphatidylcholinsekretion in das Darmlumen deutlich vermindert, welches in Inkubationsexperimenten mit Biopsieproben bewiesen wurde. Somit wird ferner der Nachweis von MDR3-analogen Proteinen zur Diagnose der Colitis ulcerosa im terminalen Ileum und Pouchepithel beschrieben. Dies beinhaltet auch alle Erkrankungen, die mit verminderter Präsenz des MDR3-analogen Proteins einhergehen.

Aus der Korrelation zwischen der Konzentration oder Menge an MDR3-analogen Proteinen können auch Rückschlüsse auf die erforderliche optimale Menge an Phosphatidylcholin im Laufe der Therapie mit den erfindungsgemäßen Arzneimitteln gefolgert werden. Die Diagnose ermöglicht so eine individuelle Anpassung der erforderlichen Dosis an Phosphatidylcholin der zu behandelnden Patienten. Außerdem ermöglicht die Bestimmung des Phosphatidylcholins im Darmlumen eine unter dem Aspekt der Patientencompliance wichtige Verlaufskontrolle in der Therapie.

Die Bestimmung der MDR3-analogen Proteine erfolgt indirekt über die RT-PCR-Vermehrung der für diese Proteine codierenden RNA (genetische Information).

Das MDR3 Protein bzw. die MDR3 Proteine sind Phospholipidtransporter in Plasmamembranen. Die menschliche MDR (P-Glykoprotein) Genfamilie besteht aus zwei Mitgliedern (MDR1 und MDR3). Sie gehören entwicklungsgeschichtlich zur sehr alten Superfamilie der ABC-Transporter (ATP-Binding-Cassette). Diese Proteine transportieren unterschiedlichste Substrate (hydrophobe Moleküle, Oxyanionen, Cl⁻ u.a.) von der Innenseite der Zelle über die Plasmamembran nach außen unter Aufwendung von Energie (ATP). Die beiden homologen MDR Gene liegen im Genom hintereinander (MDR3 ca. 30 kb downstream von MDR1) und umfassen zusammen etwa 230 kb. Beide Gene bestehen aus jeweils 28 Exons, von denen 27 beim MDR3 die Kodierregion enthalten. Vom MDR3 Gen sind vier Splicevarianten bekannt, von denen jede das Leseraster intakt läßt. In Variante C⁻¹⁴¹ fehlt das gesamte Exon 23 und führt so zu einer um 141 Nukleotide verkürzten Kodierregion. Exon 23 enthält die Transmembrandomäne 11. Ein Fehlen könnte zu einem Protein mit völlig unterschiedlicher Membrantopologie führen. In MDR3 exprimierenden Geweben sind das normale Transkript und dessen Varianten meist gleich abundant vertreten.

Das Produkt des MDR1-Gens ist verantwortlich für die Resistenz gegen verschiedene Cytostatika und gab der Familie ihren Namen: multidrug resistance (MDR). MDR3 ist eine membranständige, 170 kD große, energieabhängige Effluxpumpe für verschiedenste liphopile Substanzen. Wie bei anderen Mitgliedern der ABC-Transporter besteht MDR1 aus zwei homologen Hälften, jede mit sechs transmembranen Domänen und einer Nukleotid-Bindungsstelle. MDR3 ist strukturell genauso aufgebaut, hat aber sowohl ein anderes gewebespezifisches Verteilungsmuster als auch eine andere Funktion, da es nicht zur "multidrug resistance" führt. In Mäusen, in welchen das entsprechende Maus-spezifische Gen (mdr2) ausgeschaltet wurde (knock-out-Mausmodell), fand sich eine vollständige Unfähigkeit der Leber, Phosphatidylcholin in die Galle zu sezernieren. Man schloß daraus, daß auch MDR3 P-Glycoprotein für den Transport von Phosphatidylcholin aus der Leberzelle in die Galle verantwortlich ist. Über das Vorkommen von MDR3 im übrigen Gastrointestinaltrakt wurde bisher in der Literatur nicht berichtet.

### Beispiel 1

### Bestimmung von MDR3-analogen Proteinen über MDR3-RNA im Gastrointestinaltrakt mittels RT-PCR

Zur Untersuchung einer möglichen Expression wurden Primer zur Amplifikation von MDR3-spezifischen Transkripten synthetisiert. Ziel des Primerdesigns ist es, das gewünschte DNA-Segment spezifisch und effizient zu amplifizieren. Zur Gewährleistung der Spezifität werden die Primersequenzen aus Bereichen der MDR3-cDNA-Sequenz gewählt, in denen MDR3 und MDR1 eine geringe Homologie aufweisen. Dabei sind Primer, die eine hohe Homologie zu MDR1 aufweisen, deren 3'-terminale Base sich jedoch von der MDR1-cDNA-Sequenz unterscheidet, auch MDR3-spezifisch. Um eine effiziente Amplifizierung des gewünschten DNA-Fragmentes sicherzustellen, müssen weitere Faktoren berücksichtigt werden. Die Primersequenz muß so gewählt werden, daß die Primer nicht in der Lage sind, über intramolekulare Wasserstoffbrückenbindungen Schleifenstrukturen oder über intermolekulare Bindungen Dimere zu bilden. Die Oligonukleotidlänge beträgt vorzugsweise 20 bis 25 Basenpaare. Der GC-Gehalt sollte im Bereich von 50 bis 60 % liegen. Die beiden Primer eines Primerpaares sollen nach Möglichkeit in verschiedenen Exons liegen, damit eine Amplifizierung genomischer DNA bei der PCR ausgeschlossen bzw. erkannt werden kann.

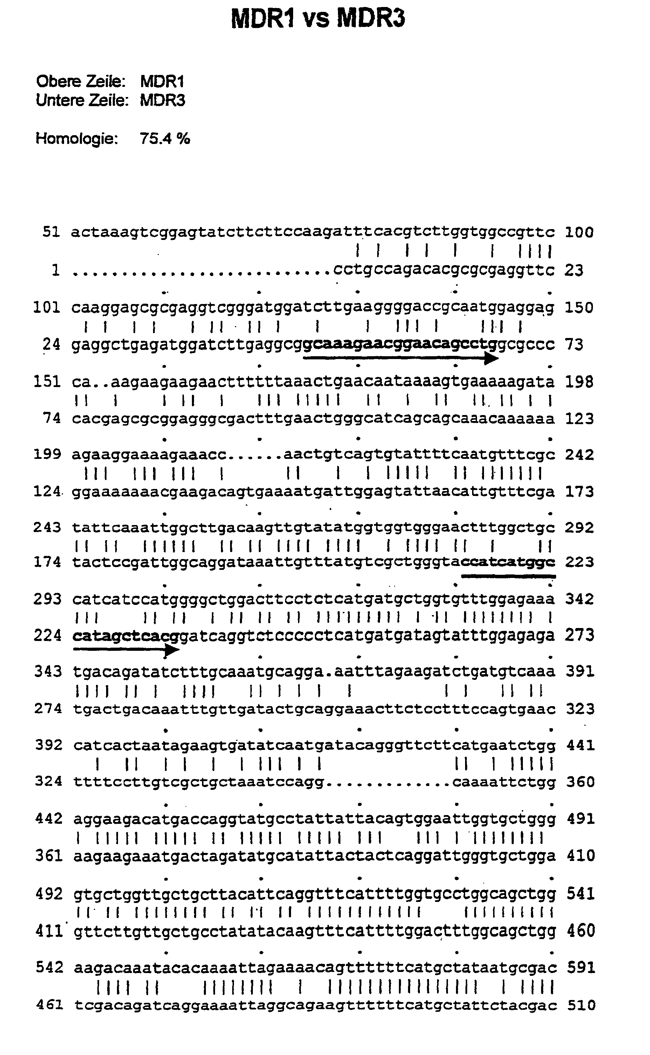

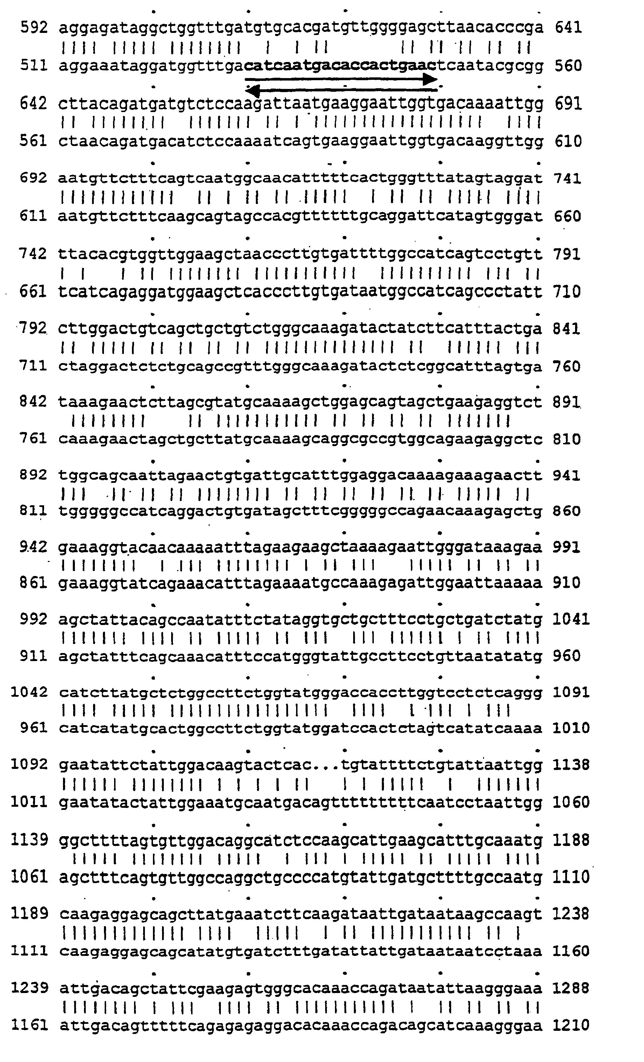

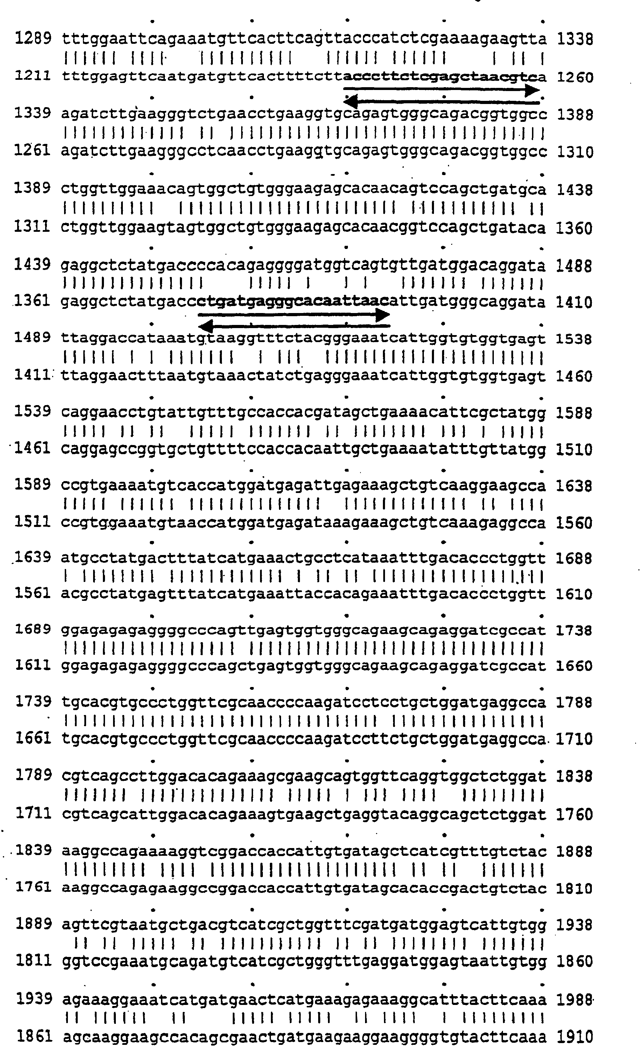

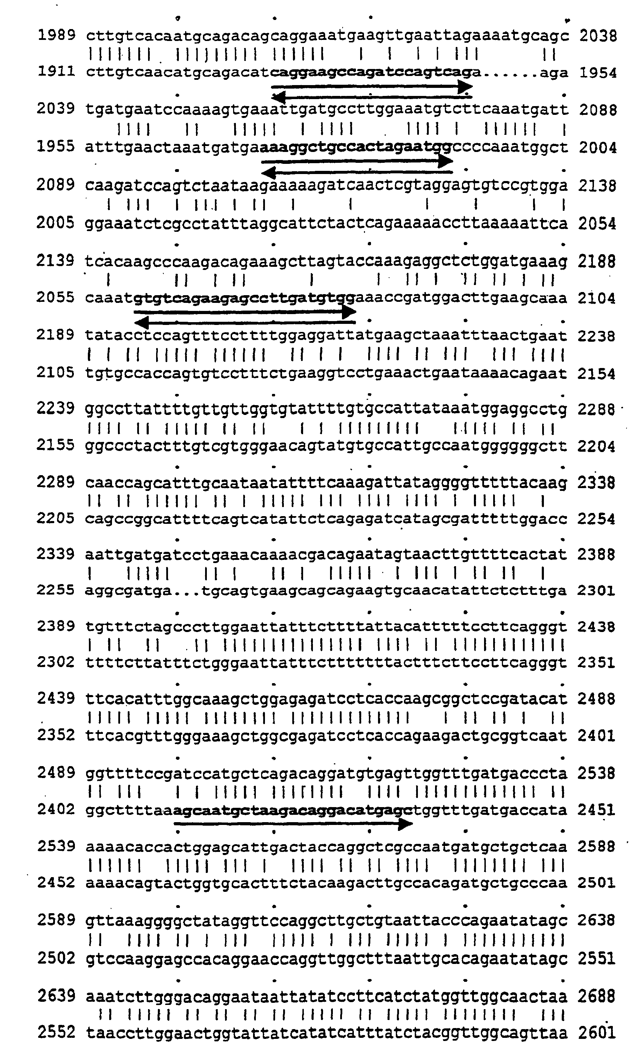

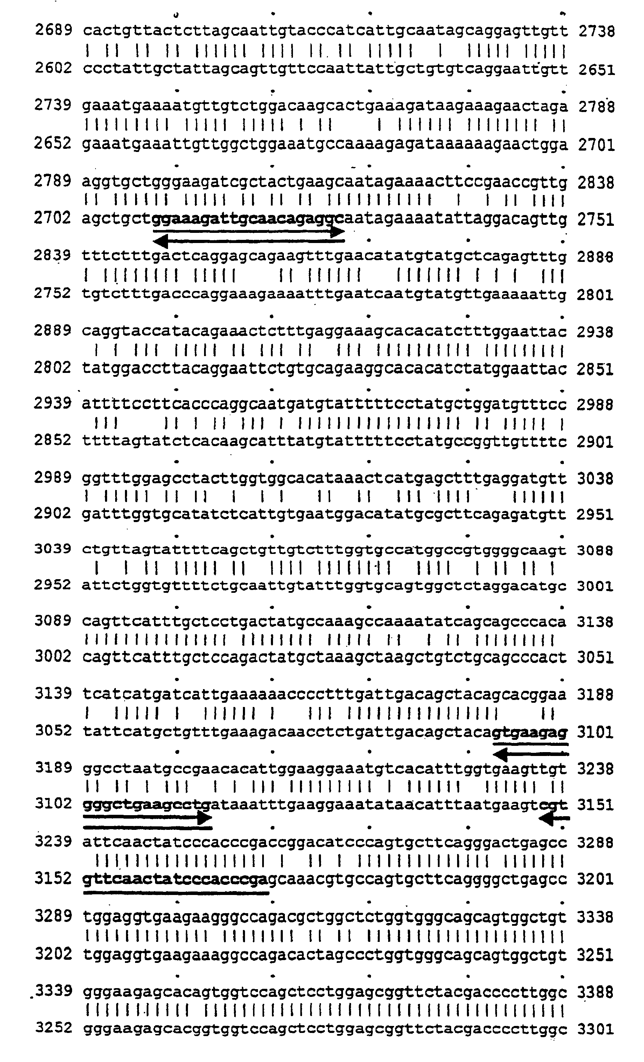

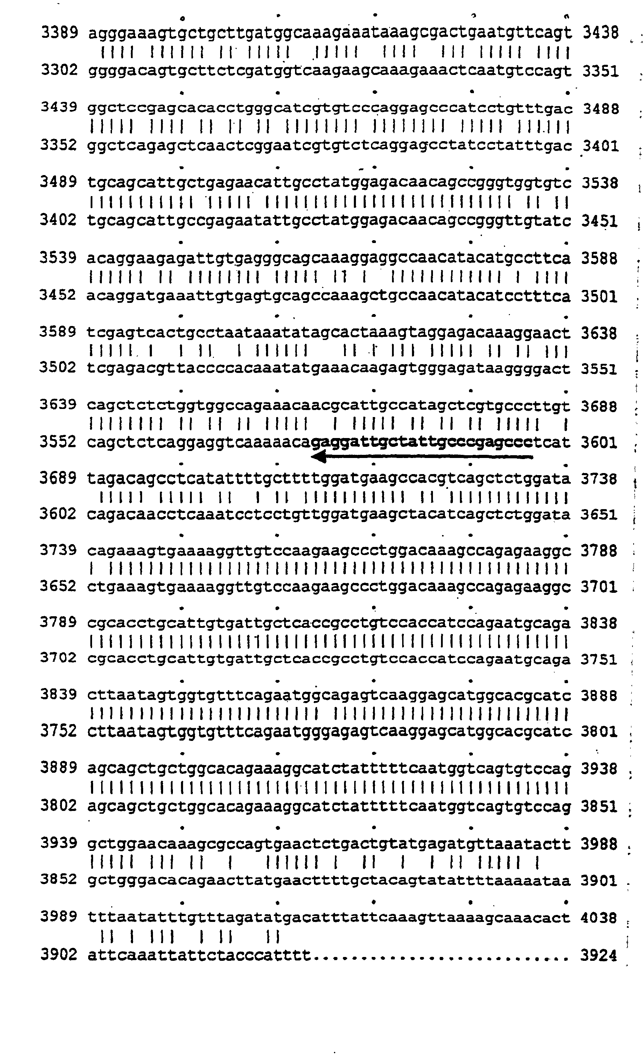

Es wurden Primersequenzen ausgewählt, die eine zuverlässige und spezifische Amplifikation von MDR3-Sequenzen ermöglichen. Beispiele geeigneter Primer, die spezifisch für MDR3 sind, sind in der beigefügten Abbildung (Abb. 1, MDR1 vs MDR3) mit Pfeilen entsprechend gekennzeichnet.

### Legende zu Abb. 1:

Homologie zwischen den cDNA-Sequenzen von MDR1 und MDR3. Die obere Zeile zeigt jeweils die MDR1-, die untere die MDR3-Sequenz. Identische Basen sind durch senkrechte Striche zwischen den Sequenzen markiert. Sequenzen, von denen Primer abgeleitet werden können, sind fett gedruckt; die Orientierung der Primer ist durch Pfeile gekennzeichnet (5'---->3' bzw. 3'<-----5').

### Beispiel einer repräsentativen, MDR3 spezifischen PCR:

Als 5'-Primer wird ein 25 bp großes Oligonukleotid von bp 2411-2435 der cDNA im Exon 20 ausgewählt. Der 3'-Primer ist ein 33 bp großes Oligonukleotid von bp 3180-3148 und liegt im Exon 25. Das entstehende PCR-Produkt ist damit 769 bp groß. Mit diesem Primerpaar kann auch die Splicevariante C⁻¹⁴¹ in der RNA nachgewiesen werden, da hier das Exon 23 fehlt und so das entstehende PCR-Produkt um 141 Basenpaare verkürzt ist. Als Ausgangsmaterial für die RNA-lsolierung werden Biopsien verwendet, die bei Gastroskopien bzw. Koloskopien anfielen. Dabei werden jeweils 2 Biopsien eines Patienten zur Isolierung von RNA mit dem Fast-RNA Kit der Firma Dianova verwendet. Die Mengen an Gesamt RNA, die damit isoliert werden kann, beträgt bis zu 70 µg. Auf eine Isolierung von Poly(A⁺)RNA kann verzichtet werden, da die Menge an Transkript in der Gesamt-RNA bereits für einen PCR-Nachweis ausreichend ist.

5 µg Gesamt-RNA wird für eine Erststrang cDNA-Synthese eingesetzt. Die cDNA-Synthese und die PCR erfolgen mit dem ReadyToGo-System (Pharmacia) nach den Empfehlungen des Herstellers. 5 µg Gesamt-RNA werden revers in cDNA transkribiert. Die PCR wird mit 2 von 33 µl cDNA und 10 pmol von jedem Primer in einem Gesamtvolumen von 25 µl angesetzt. Die Amplifizierung wird wie folgt durchgeführt: Einmal 5 Minuten bei 94 °C, daran anschließend 33 Zyklen von 2 sec bei 94 °C , 2 sec bei 55 °C und 45 sec bei 72 °C. Die RT-PCR-Produkte werden durch Agarosegelelektrophorese analysiert. Die Bedingungen für einen erfolgreichen Nachweis von MDR3 werden mit Leber-RNA ausgetestet, da hier eine besonders hohe Abundanz an Transkripten vorliegt. Als interner Standard wird GAPDH amplifiziert, um die Menge an cDNA in den verschiedenen Ansätzen vergleichen zu können. Die eingesetzte Methodik eignet sich auch für die "Light Cycler"-Technologie (Roche Diagnostics GmbH oder die "Taq Man"-PCR Technologie (Perkin Elmer Applied Bio-Systems)), bei der eine große Anzahl von Proben gleichzeitig, schnell und (semi)quantitativ bestimmt werden kann.

Es werden Biopsien aus Ösophagus, Magen, Duodenum, terminalem lleum, Colon transversum und Rektum untersucht. Bei gesunden Patienten konnten MDR3-Transkripte in Magen, Duodenum und terminalem lleum nachgewiesen werden, während sie im Ösophagus, Colon transversum und Rektum fehlten.

Der oben genannte Ansatz wird auch immer und in gleicher Menge die Splicevariante C⁻¹⁴¹ mitamplifziert. Die beiden PCR-Produkte wurden dann im puc18 kloniert und ansequenziert. Dadurch konnte dokumentiert werden, daß es sich bei den amplifizierten Sequenzen wirklich um MDR3 handelt. Grundsätzlich ist es nach der oben beschriebenen Methode auch möglich, außer dem organspezifischen MDR3-analogen Protein andere homologe Proteine im Ileum nachzuweisen, wobei es sich bei diesen anderen homologen Proteinen beispielsweise um Isoformen des MDR3-analogen Proteins (z.B. Isoform des Leber oder Darm MDR3-Proteins) handeln kann.

### Beispiel 2

### Expression von MDR3-analogen Proteinen (RNA) im ileoanalen Pouchepithel

Untersucht wurden Patienten, die aufgrund einer fortgeschrittenen Colitis ulcerosa colektomiert werden mußten und einen Pouch erhielten. Bei keinem dieser Colitis ulcerosa Patienten konnten in der Pouchschleimhaut MDR3-analoge Transkripte nachgewiesen werden. Es stellte sich deshalb die Frage, ob das Fehlen der Transkripte auf die Tatsache zurückzuführen war, daß es sich um Colitis ulcerosa Patienten handelte. Denkbar wäre auch, daß mit Anlegen eines Pouches per se und des damit einhergehenden veränderten bakteriellen Milieus für das terminale lleum ein verändertes Expressionsmuster aufgetreten sein könnte. Deshalb wurden im Vergleich Patienten untersucht, die einen Pouch nach Colektomie bei familiärer adenomatöse Polyposis (FAP) erhielten. Diese Patienten wiesen MDR3-Transkripte im Pouch auf. Dies wies darauf hin, daß das Fehlen von MDR3 im Pouch spezifisch für die Colitis ulcerosa ist. Die dadurch möglicherweise bedingte verminderte Phosphatidylcholinsekretion in den Schleim könnte somit die beobachtete Pouchitis bahnen. Da die Pouchschleimhaut der des terminalen lleums entspricht, müßte folgerichtig bei Colitis ulcerosa auch in diesem Dünndarmabschnitt MDR3 fehlen.

### Beipiel 3

### Expression von MDR3-analogen Proteinen (RNA) im terminalen lleum bei Gesunden, Patienten mit Morbus Crohn und Colitis ulcerosa

Es wurde untersucht, ob Colitis ulcerosa Patienten im terminalen lleum eine MDR3-analoge Expression zeigen. Analysiert wurden 31 Patienten mit Colitis ulcerosa und verglichen mit 14 Gesunden und 6 Morbus Crohn Patienten.

Bei keinem der Colitis ulcerosa Patienten konnte mit der verwendeten, hoch sensitiven Technik MDR3-analoge RNA im terminalen Ileum nachgewiesen werden, während es bei allen gesunden Kontrollpersonen und bei den Morbus Crohn Patienten nachweisbar war. Dieses eindeutige Ergebnis weist darauf hin, daß ausschließlich bei Colitis ulcerosa ein Defekt in der Expression des MDR3-Analogons vorliegt. Daraus kann geschlossen werden, daß bei diesen Patienten eine gestörte Phospholipidsekretion in den Darm vorliegt, welches die Schleimzusammensetzung so stört, daß Bakterien das Epithel eher angreifen können.

## Patentansprüche

1. Verwendung von Phosphatidycholin zur Herstellung von Arzneimitteln mit schleimhautschützender Wirkung im Dickdarm zur rektalen Verabreichung oder oralen Verabreichung mit verzögerter Wirkstofffreisetzung im unteren Ileum oder Colon zur Behandlung von Colitis ulcerosa, Pouchitis, Dickdarmerkrankungen oder Entzündungen im Dickdarm wie Morbus Crohn, Diversionscolitis, infektiöse Enteritis/Colitis, Entzündungen durch Bestrahlung, Antibiotika, Chemotherapeutica, Pharmaka oder Chemikalien, oder zur Behandlung oder Prophylaxe des Dickdarmcarcinoms.

2. Verwendung nach Anspruch 1, **dadurch gekennzeichnet, daß** die rektale Verabreichung zur lokalen Behandlung von Entzündungen im Rektum oder Pouch erfolgt.

3. Verwendung nach Anspruch 1, **dadurch gekennzeichnet, daß** die orale Applikation mit einem Wirkstoffgehalt von 1 - 500 mg pro Fertigarzneiform erfolgt.

## Claims

1. Use of phosphatidylcholine for the manufacture of medicaments having mucosa-protecting action in the large intestine for rectal administration or oral administration with delayed active ingredient release in the lower ileum or colon for the treatment of ulcerative colitis, pouchitis, diseases of the large intestine or inflammation in the large intestine, such as Crohn's disease, diversion colitis, infectious enteritis/colitis, inflammation caused by irradiation, antibiotics, chemotherapeutic agents, drugs or chemicals, or for the treatment or prophylaxis of carcinoma of the large intestine.

2. Use according to claim 1, **characterised in that** the rectal administration is effected for the local treatment of inflammation in the rectum or pouch.

3. Use according to claim 1, **characterised in that** the oral application is effected with an active ingredient content of 1-500 mg per finished medicament form.

## Revendications

1. Utilisation de phosphatidylcholine pour la fabrication de médicaments à action de protection des muqueuses dans le gros intestin pour l'administration rectale ou l'administration orale avec libération accélérée du principe actif dans l'intestin inférieur ou côlon pour le traitement de Colitis ulcerosa, Pouchitis, des maladies du gros intestin ou des irritations dans le gros intestin comme Morbus Crohn, Diversions-colistis, Enteritis/Colitis infectieux, irritations par irradiation, antibiotiques, chimiothérapie, produits pharmaceutiques ou produits chimiques ou pour le traitement ou la prophylaxie du carcinome du gros intestin.

2. Utilisation selon la revendication 1,
**caractérisée en ce que** l'administration rectale pour le traitement local d'irritations est effectuée dans le rectum ou la poche.

3. Utilisation selon la revendication 1,
**caractérisée en ce que** l'application orale est effectuée avec une teneur en principe actif de 1-500 mg par forme médicamenteuse finie.
